(19) Europäisches Patentamt
European Patent Office
Office européen des brevets

(11) EP 3 677 268 A1

(12) EUROPEAN PATENT APPLICATION
published in accordance with Art. 153(4) EPC

(43) Date of publication:
**08.07.2020 Bulletin 2020/28**

(21) Application number: **18851853.4**

(22) Date of filing: **30.08.2018**

(51) Int Cl.:
***A61K 31/713** (2006.01)* ***A61K 9/10** (2006.01)*
***A61K 47/34** (2017.01)* ***A61K 48/00** (2006.01)*
***A61P 35/00** (2006.01)* ***C12N 15/09** (2006.01)*

(86) International application number:
**PCT/JP2018/032040**

(87) International publication number:
**WO 2019/044937 (07.03.2019 Gazette 2019/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.08.2017 JP 2017166735**

(71) Applicants:
- **The University Of Tokyo**
**Tokyo 113-8654 (JP)**
- **Kawasaki Institute of Industrial Promotion**
**Kawasaki-shi, Kanagawa 212-0013 (JP)**

(72) Inventors:
- **KATAOKA, Kazunori**
**Kawasaki-shi**
**Kanagawa 212-0013 (JP)**
- **MIYATA, Kanjiro**
**Tokyo 113-8654 (JP)**
- **FUKUSHIMA, Shigeto**
**Kawasaki-shi**
**Kanagawa 212-0013 (JP)**
- **HAYASHI, Kotaro**
**Kawasaki-shi**
**Kanagawa 212-0013 (JP)**
- **WATANABE, Sumiyo**
**Tokyo 113-8654 (JP)**
- **KIM, HyunJin**
**Tokyo 113-8654 (JP)**
- **TOH, Kazuko**
**Kawasaki-shi**
**Kanagawa 212-0013 (JP)**

(74) Representative: **Müller-Boré & Partner Patentanwälte PartG mbB**
**Friedenheimer Brücke 21**
**80639 München (DE)**

(54) **NUCLEIC ACID-LOADED UNIT POLYION COMPLEX**

(57) The present invention provides a unit-type polyion complex, comprising: a specific number of molecules of a block copolymer having a poly(ethylene glycol) segment and a cationic poly(amino acid) segment; and a specific number of molecules of a nucleic acid, wherein a total positive charge quantity derived from side chains of the cationic poly(amino acid) segment of the block copolymer in the unit-type polyion complex is prevented from being counterbalanced with a total negative charge quantity derived from the nucleic acid, wherein the nucleic acid has a strand length of from 10-base length to 50-base length, wherein the poly(ethylene glycol) segment has a molecular weight of $40 \times 10^3$ or more, and wherein the block copolymer has a binding constant ($K_a$) for the nucleic acid of $3.0 \times 10^5$ or more.

FIG. 1

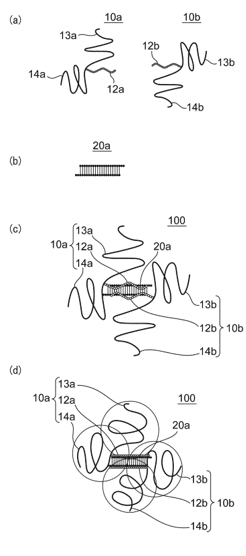


EP 3 677 268 A1

## Description

Technical Field

The present invention relates to a nucleic acid-loaded unit-type polyion complex and a composition including the unit-type polyion complex.

Background Art

Nucleic acid therapeutics can regulate various genes in cells, and hence are highly expected to be applied to various diseases, such as cancer and genetic disorders, which have heretofore been considered difficult to treat. Meanwhile, the nucleic acid therapeutics require an appropriate technology for delivery thereof to target cells because of low *in vivo* stability of a nucleic acid molecule. For example, as the delivery technology for a nucleic acid, hitherto, there has been known a technology utilizing a block copolymer having a hydrophilic polymer segment and a cationic polymer segment to form a complex with a nucleic acid via an electrostatic interaction (polyion complex).

Examples of the polyion complex to be obtained in the hitherto known technology include: a polyion complex formed of an unspecified number of nucleic acids and an unspecified number of block copolymers, the block copolymers being radially arranged with their hydrophilic polymer segments directed outward and cationic polymer segments directed inward in a state of encapsulating the nucleic acids (hereinafter sometimes referred to as "micelle-type polyion complex") ; and a polyion complex formed of a specific number of nucleic acids and a specific number of block copolymers, the polyion complex existing as a stable unit structure under *in vivo* conditions, for example, bloodstream (hereinafter sometimes referred to as "unit-type polyion complex" or "uPIC") (for example,

Patent Literature 1).

The unit-type polyion complex described in Patent Literature 1 has a definite structure formed of a specific number of nucleic acids and a specific number of block copolymers, and can improve blood retention property of the nucleic acids.

Citation List

Patent Literature

[PTL 1] WO 2013/162041 A1

Summary of Invention

Technical Problem

In the preparation of the unit-type polyion complex described in Patent Literature 1, the design of the nucleic acids and/or the block copolymers needs to be carefully performed so that negative charge derived from the nucleic acids and positive charge derived from the block copolymers are counterbalanced to make the unit-type polyion complex electrically neutral.

The invention of the present application has been made in order to solve the above-mentioned problem, and a primary object of the invention of the present application is to provide a unit-type polyion complex that can be designed more simply.

Solution to Problem

According to one embodiment of the present invention, there is provided a unit-type polyion complex, including: a specific number of molecules of a block copolymer having a poly(ethylene glycol) segment and a cationic poly(amino acid) segment; and a specific number of molecules of a nucleic acid, wherein a total positive charge quantity derived from side chains of the cationic poly(amino acid) segment of the block copolymer in the unit-type polyion complex is prevented from being counterbalanced with a total negative charge quantity derived from the nucleic acid, wherein the nucleic acid has a strand length of from 10-base length to 50-base length, wherein the poly(ethylene glycol) segment has a molecular weight of $40\times10^3$ or more, and wherein the block copolymer has a binding constant ($K_a$) for the nucleic acid of $3.0\times10^5$ or more.

In one embodiment, the unit-type polyion complex consists of two molecules of the block copolymer and one

molecule of the nucleic acid.

In one embodiment, the nucleic acid is a double-stranded nucleic acid.

In one embodiment, the molecular weight of the poly (ethylene glycol) segment is $60\times10^3$ or more.

In one embodiment, the poly (ethylene glycol) segment is 2-chain branched, and each of poly(ethylene glycol) chains has a molecular weight of $20\times10^3$ or more.

In one embodiment, the cationic poly(amino acid) segment contains ornithine as a constituent amino acid.

According to another embodiment of the present invention, there is provided a composition for nucleic acid delivery, including: the unit-type polyion complex; and a block copolymer capable of forming the unit-type polyion complex, the block copolymer being in a free state.

In one embodiment, a ratio (N/P ratio) between a molar concentration of amino groups derived from the block copolymer and a molar concentration of phosphate groups derived from the nucleic acid in the composition is from 1 to 20.

Advantageous Effects of Invention

In the present invention, the block copolymer having the predetermined affinity for the nucleic acid is used. As a result, surprisingly, the unit-type polyion complex that can improve the blood retention property of the nucleic acid even in a state in which the charges are not counterbalanced with each other is obtained. Such a unit-type polyion complex does not require strict control of the negative charge derived from the nucleic acid and the positive charge derived from the block copolymer upon preparation, and hence can be designed more simply than the related-art unit-type polyion complex.

Brief Description of Drawings

FIGS. 1 are schematic views for illustrating the structure of a unit-type polyion complex according to one embodiment of the present invention.

FIG. 2 is a graph for showing the average particle diameter of a unit-type polyion complex and a nucleic acid association number in the unit-type polyion complex.

FIG. 3 is a graph for showing the blood retention property of a nucleic acid.

Description of Embodiments

[A. Unit-type Polyion Complex]

According to the present invention, there is provided a unit-type polyion complex including: a specific number of molecules of a block copolymer having a poly(ethylene glycol) segment and a cationic poly(amino acid) segment; and a specific number of molecules of a nucleic acid, wherein a total positive charge quantity derived from side chains of the cationic poly(amino acid) segment of the block copolymer in the unit-type polyion complex is prevented from being counterbalanced with a total negative charge quantity derived from the nucleic acid (as a result, the unit-type polyion complex is not electrically neutralized). Embodiments of the present invention are described below, but the present invention is not limited to these embodiments. The block copolymer and the nucleic acid to be used in the present invention may each exhibit a certain degree of polydispersity when obtained as a polymerization reaction product. Therefore, herein, when a mention is made about properties (such as molecular weight, polymerization degree, and charge) of the block copolymer and properties (such as base length and charge) of the nucleic acid, the mention is made about the average of the whole polymerization reaction product exhibiting polydispersity, unless otherwise specified. Therefore, for example, the charge quantity of the block copolymer is calculated on the basis of an average polymerization degree through the use of the polymerization degree of cationic amino acid residues obtained by actual measurement as the average polymerization degree. In addition, for example, an average molecular weight (e.g., number-average molecular weight) obtained by actual measurement may be applied as the molecular weight of the poly (ethylene glycol) segment.

[A-1. First Embodiment]

[A-1-1. Entire Structure of Unit-type Polyion Complex]

FIG. **1(a)** to FIG. **1(d)** are schematic views for illustrating an example of a uPIC according to a first embodiment of the present invention. Specifically, FIG. **1(a)** is a schematic view of block copolymers that may be used in the present invention, FIG. **1(b)** is a schematic view of a nucleic acid that may be used in the present invention, FIG. **1(c)** is a schematic view of a uPIC consisting of the block copolymers of FIG. **1(a)** and the nucleic acid of FIG. **1(b)**, and FIG. **1(d)** is a schematic view for illustrating the spatial extent of PEG segments in the uPIC of FIG. **1(c).**

Block copolymers **10a** and **10b** illustrated in FIG. **1(a)** have 2-chain branched PEG segments **13a** and **14a,** and **13b** and **14b,** respectively, and cationic poly(amino acid) segments **12a and 12b,** respectively. In addition, a nucleic acid **20a** illustrated in FIG. **1(b)** is a double-stranded nucleic acid having an overhang of two bases at each end. The block copolymers **10a** and **10b,** and the nucleic acid **20a** associate with each other through electrostatic interactions between positive charges derived from the cationic poly (amino acid) segments **12a** and **12b** and negative charges derived from the nucleic acid **20a,** to thereby form a uPIC **100** (FIG. **1(c)).** In the uPIC **100,** the block copolymers **10a** and **10b** may be typically arranged opposite to each other in the main chain direction of the nucleic acid **20a.** With this, steric hindrance due to mutual overlapping of the PEG segments **13a, 13b, 14a,** and **14b** can be suppressed. As a result, even when having PEG segments that are branched and have long chains, the block copolymer can suitably form electrostatic bonds with the nucleic acid to form the uPIC. In addition, with such arrangement, as illustrated in FIG. **1(d),** the four PEG segments **13a, 13b, 14a,** and **14b** can extend so as to entirely cover the nucleic acid **20a,** thereby being able to contribute to a high level of protection of the nucleic acid **20a.** In one embodiment, the four PEG segments may be located so that a terminus of each of the PEG segments serves as a vertex of an approximately regular tetrahedron, and the nucleic acid may be located in the regular tetrahedron.

The uPIC according to this embodiment only needs to include a specific number of molecules of the block copolymer and a specific number of molecules of the nucleic acid, and is not limited to the structure of the illustrated example.

The uPIC according to this embodiment is such that, in a physiological pH environment (pH = about 7.4), a positive charge quantity derived from side chains of the cationic poly (amino acid) segment of the block copolymer is prevented from being counterbalanced with a negative charge quantity derived from the nucleicacid, and as a result, the uPIC is not electrically neutralized. Herein, the description "a total positive charge quantity derived from side chains of the cationic poly(amino acid) segment of the block copolymer in the unit-type polyion complex is prevented from being counterbalanced with a total negative charge quantity derived from the nucleic acid" or "the uPIC is not electrically neutralized" means that the total charge quantity (absolute value) derived from the side chains of the cationic poly(amino acid) segments of all block copolymer molecules contained in the uPIC is less than 90% or more than 110% of the total charge quantity (absolute value) derived from all nucleic acid molecules contained in the uPIC. In the development of a uPIC, hitherto, in the case where positive charges derived from the side chains of the cationic poly(amino acid) segment and negative charges derived from the nucleic acid are not counterbalanced with each other, there has been a concern that the uPIC may be brought into a charged state as a whole to electrostatically attract a protein or enzyme in blood, resulting in a reduction of blood retention property, or the charged uPICs may further associate with each other or with the block copolymer and/or the nucleic acid, resulting in a reduction in uniformity. However, according to an investigation made by the inventors of the present invention, it has been found that even a charged uPIC can be allowed to stably maintain its structure to achieve excellent blood retention property by selecting a block copolymer having a predetermined affinity for the nucleic acid and adopting a long PEG segment. In one embodiment, the total positive charge quantity derived from the side chains of the cationic poly (amino acid) segments of all block copolymer molecules in the uPIC in a physiological pH environment may be 45% or more and less than 90% (e.g., from 50% to 85%, or from 50% to 80%) of the total negative charge quantity derived from all nucleic acid molecules in the uPIC. In another embodiment, the total positive charge quantity derived from the side chains of the cationic poly(amino acid) segments of the block copolymer in the uPIC in a physiological pH environment may be more than 110% and 150% or less (e.g., from 115% to 140%) of the total negative charge quantity derived from the nucleic acid.

The uPIC according to this embodiment typically has a smaller size than a micelle-type PIC. The particle diameter (hydrodynamic diameter) of the uPIC according to this embodiment is preferably from 10 nm to 30 nm, and may be, for example, from 10 nm to 25 nm, or for example, from 10 nm to 20 nm. Such small size can contribute to delivering the loaded nucleic acid to a deep part of a tissue.

[A-1-2. Block Copolymer]

The block copolymer that may be used in this embodiment has the PEG segment and the cationic poly(amino acid) segment, and has a binding constant ($K_a$) of $3.0\times10^5$ or more, preferably $5.0\times10^5$ or more (e.g., $8.0\times10^5$ or more,

$1.0\times10^6$ or more, $1.5\times10^6$ or more, or $2.0\times10^6$ or more) for the nucleic acid to be delivered. When the block copolymer having the above-mentioned predetermined affinity for the nucleic acid is used, in blood, the block copolymer is hardly dissociated from the uPIC, or can be quickly reassociated even when dissociated. Presumably as a result of this, even under a state in which the negative charges derived from the nucleic acid and the positive charges derived from the block copolymer are not counterbalanced with each other, the nucleic acid can be satisfactorily protected to achieve high blood retention property. Meanwhile, the upper limit value of the binding constant ($K_a$) is not particularly limited, but from the viewpoint of the ease of achievement, may be, for example, $4.0\times10^6$ or less. Herein, the block copolymer encompasses a pharmaceutically acceptable salt of the block copolymer.

The binding constant ($K_a$) is measured by a method described later in Examples. The binding constant ($K_a$) tends to increase as the number of electrostatic bonds between the cationic poly(amino acid) segment and the nucleic acid increases or as individual electrostatic bonds become stronger. Accordingly, the binding constant ($K_a$) may be controlled to fall within a desired range by, for example, adjusting the positive charge number of the cationic poly(amino acid) segment, or appropriately selecting the kinds of constituent amino acids of the cationic poly(amino acid) segment. When the structure of the block copolymer is fixed, it is considered that the binding constant ($K_a$) depends mainly on the negative charge quantity of the nucleic acid and does not substantially change depending on a difference in base sequence for the same base length.

The PEG segment is preferably branched, more preferably 2-chain branched. Needless to say, the PEG segment may be of a single-chain type including only one PEG chain.

The molecular weight of the PEG segment (when the PEG segment is branched, the total of the molecular weights of respective PEG chains) is $40\times10^3$ or more, preferably $50\times10^3$ or more, more preferably $60\times10^3$ or more. When the PEG segment is branched, the molecular weights of the respective PEG chains are not limited as long as the total of the molecular weights of the respective chains satisfies the above-mentioned range, and may be the same or different from each other. The molecular weight of each of the PEG chains of the branched PEG segment may be preferably $10\times10^3$ or more, for example, $20\times10^3$ or more, $25\times10^3$ or more, $30\times10^3$ or more, or $35\times10^3$ or more. It is preferred that each of the PEG chains of the branched PEG segment has a molecular weight of $20\times10^3$ or more, $25\times10^3$ or more, $30\times10^3$ or more, or $35\times10^3$ or more. Meanwhile, the molecular weights of the respective PEG chains may be independently, for example, $80\times10^3$ or less, $60\times10^3$ or less, or $50\times10^3$ or less. With the molecular weight of the PEG segment falling within such range, even when the uPIC is in a charged state, the nucleic acid can be suitably protected from enzymatic degradation and the like. In addition, the biocompatibility of the uPIC can be enhanced to prevent its decomposition or elimination as foreign matter. The molecular weight of the PEG segment may be determined by gel filtration chromatography (GFC) or the like, and in the determination, commercially available PEG having a known molecular weight (e.g., PEG for use as a molecular weight standard for GFC) may be used as a molecular weight marker.

The cationic poly(amino acid) segment may contain any appropriate basic amino acid as a constituent unit as long as the cationic poly(amino acid) segment has a desired positive charge in a physiological pH environment and the binding constant ($K_a$) of the block copolymer for the nucleic acid is $3.0\times10^5$ or more. The basic amino acid is preferably at least one kind of basic amino acid selected from lysine, ornithine, 2,3-diaminopropionic acid, and 2,4-diaminobutyric acid because a desired binding constant can be easily achieved. Of those, ornithine is more preferred.

The ratio of the number of the at least one kind of basic amino acids (when two or more kinds of the basic amino acids are contained, the total number thereof) to the number of all amino acid residues constituting the cationic poly(amino acid) segment may be preferably from 50% to 100%, and may be, for example, from 80% to 100%, from 90% to 100%, or 100%.

The cationic poly(amino acid) segment may further contain any other basic amino acid as a constituent unit as long as a desired binding constant is obtained. Examples of the other basic amino acid include natural basic amino acids, such as arginine and histidine, and amino acid derivatives obtained by introducing a cationic residue into a side chain of an acidic amino acid (e.g., Asp(DET) obtained by introducing an ethylenediamine structure into a side chain of aspartic acid).

The positive charge quantity derived from the side chains of the cationic poly (amino acid) segment (per molecule of the block copolymer) may be appropriately set depending on, for example, a desired binding constant ($K_a$) for the nucleic acid, a structure of the uPIC, a negative charge quantity of the nucleic acid, and a kind of basic amino acid residue. The suitable range of the positive charge quantity (per molecule of the block copolymer) may vary depending on, for example, the negative charge quantity of the nucleic acid and the kind of the basic amino acid residue, and hence the positive charge quantity is not limited, but may be an integer of, for example, 9 or more, 10 or more, or 11 or more. From a different viewpoint, the positive charge quantity (per molecule of the block copolymer), which is similarly not limited, may be 22.5% or more, 25% or more, or 27% or more of the negative charge quantity of the nucleic acid. When such positive charge quantity is achieved through the use of the basic amino acid residue (e.g., lysine, ornithine, 2,3-diaminopropionic acid, or 2,4-diaminobutyric acid), a block copolymer having a binding constant ($K_a$) equal to or higher than a predetermined value for the nucleic acid can be suitably obtained. Meanwhile, when the positive charge quantity is excessively large, the blood retention property of the polymer itself may be reduced. Therefore, the positive charge

quantity may be preferably 50 or less, for example, 40 or less, 30 or less, or 25 or less.

The cationic poly(amino acid) segment may contain a non-basic amino acid residue (e.g., a hydrophobic amino acid residue) as long as the effect of the present invention is obtained. The number of non-basic amino acid residues may be set to, for example, 10% or less, more preferably 5% or less, still more preferably 2% or less of the number of all amino acid residues constituting the cationic poly(amino acid) segment.

The PEG segment and the cationic poly (amino acid) segment are bonded to each other via any appropriate linking group. Examples thereof include linking groups including an ester bond, an amide bond, an imino group, a carbon-carbon bond, an ether bond, and the like. In addition, these segments may be linked to each other via a linking group that is cleavable in vivo (such as a disulfide bond, a hydrazone bond, a maleamate bond, or an acetal group).

The PEG segment-side terminal and/or cationic poly(amino acid) segment-side terminal of the block copolymer may be subjected to any appropriate modification as long as the effect of the present invention is obtained. Examples of the modification include the introduction of a protective group and the introduction of a target site-binding moiety.

The target site-binding moiety may be any appropriate moiety depending on a target tissue, a purpose, or the like. The target site-binding moiety may be formed by conjugating a compound having the target site binding moiety to the free terminal of the PEG segment of the block copolymer. Herein, the target site-binding moiety refers to a moiety capable of binding specifically to substances derived from a living body and a virus to form a biological binding pair with the substances and having a biological recognition function. The introduction of the target site-binding moiety can improve the ability of the nucleic acid to arrive at a desired site serving as a target.

A preferred specific example of the block copolymer may be represented by the following general formula (1) or (2).

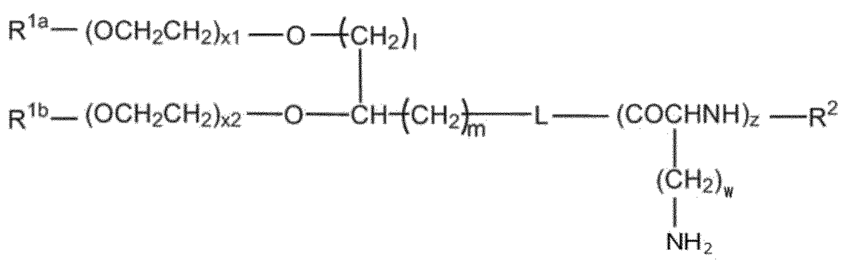

(1)

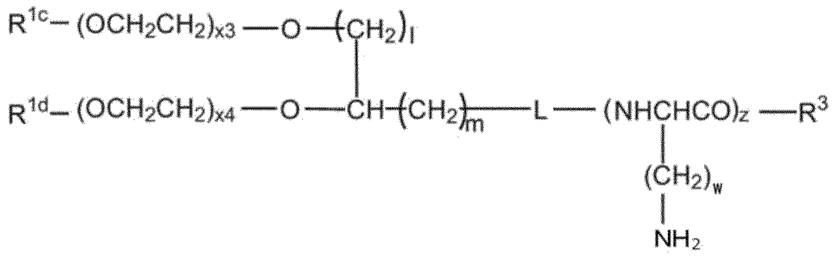

(2)

In each of the formulae,

$R^{1a}$ to $R^{1d}$ each independently represent a hydrogen atom or an unsubstituted or substituted linear or branched alkyl group having 1 to 12 carbon atoms;
$R^2$ represents a hydrogen atom, an unsubstituted or substituted linear or branched alkyl group having 1 to 12 carbon atoms, or an unsubstituted or substituted linear or branched alkylcarbonyl group having 1 to 24 carbon atoms;
$R^3$ represents a hydroxyl group, an unsubstituted or substituted linear or branched alkyloxy group having 1 to 12 carbon atoms, an unsubstituted or substituted linear or branched alkenyloxy group having 2 to 12 carbon atoms, an unsubstituted or substituted linear or branched alkynyloxy group having 2 to 12 carbon atoms, or an unsubstituted or substituted linear or branched alkyl-substituted imino group having 1 to 12 carbon atoms;
L represents a divalent linking group or a valence bond;
x1 to x4 each independently represent an integer of from 455 to 1,800;
"z" represents an integer of from 9 to 50;
"w" represents an integer of from 1 to 4; and
"1" and "m" each independently represent an integer of from 0 to 5.

In the formula (1) or (2), L represents a divalent linking group or a valence bond. Any appropriate linking group

may be adopted as the divalent linking group. For example, L may represent $-L^1-L^2-L^3-$in the formula (1) and L may represent $-L^4-L^5-L^6-$ in the formula (2). In the formulae, $L^1$ and $L^4$ each independently represent $-(O-(CH_2)_a)_b-L^{1a}-$, where "a" and "b" represent integers of from 1 to 5 and from 0 to 300, respectively, provided that it is not necessary that all "a"s be identical to each other when "b" represents 2 or more, and $L^{1a}$ represents a valence bond, -S-S-, -NH-, -O-, $-O-CH(CH_3)-O-$, -OCO-, -OCONH-, -NHCO-, -NHCOO-, -NHCONH-, -CONH-, or -COO-; $L^2$ and $L^5$ each independently represent a valence bond or $-L^{2a}-L^{2b}-L^{2c}-$, where $L^{2a}$ and $L^{2c}$ represent structures serving as spacers, an example thereof is, but not particularly limited to, a substituted or unsubstituted alkylene group having 1 to 12 carbon atoms, and $L^{2b}$ represents any one of structures represented by the following formulae (III) to (V) ; $L^3$ represents $-((CH_2)_c-O)_d-(CH_2)_e-L^{3a}-$, where "c", "d", and "e" represent integers of from 1 to 5, from 0 to 500, and from 0 to 5, respectively, provided that it is not necessary that all "c"s be identical to each other when "d" represents 2 or more; $L^{3a}$ represents -NH- or -O-; and $L^6$ represents $-((CH_2)_f-O)_g-(CH_2)_h-L^{6a}-(CH_2)_i-CO-$, where "f", "g", "h", and "i" represent integers of from 1 to 5, from 0 to 300, from 0 to 5, and from 0 to 5, respectively, provided that it is not necessary that all "f"s be identical to each other when "g" represents 2 or more, and $L^{6a}$ represents -OCO-, -NHCO-, -OCONH-, -NHCOO-, -NHCONH-, -CONH-, or -COO-.

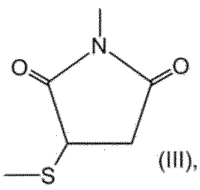 (III), 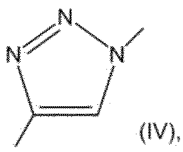 (IV), 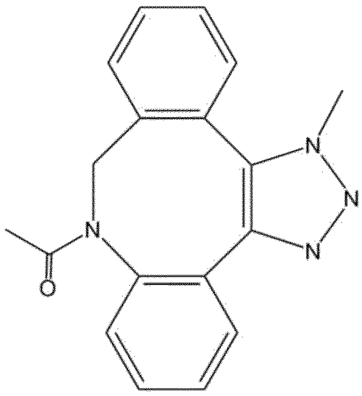 (V)

An alkyl moiety in the linear or branched alkyloxy group, alkyl-substituted imino group, and alkyl group having 1 to 12 carbon atoms, which are defined by the groups $R^{1a}$ to $R^{1d}$, $R^2$, and $R^3$, may be, for example, a methyl group, an ethyl group, a n-propyl group, an isopropyl group, a n-butyl group, a sec-butyl group, a tert-butyl group, a n-hexyl group, a decyl group, or an undecyl group. An alkenyl or alkynyl moiety in the linear or branched alkenyloxy group having 2 to 12 carbon atoms or the linear or branched alkynyloxy group having 2 to 12 carbon atoms may be exemplified by an alkenyl or alkynyl moiety including a double bond or a triple bond in the alkyl group having 2 or more carbon atoms as exemplified above.

For such group or moiety, a substituent in a "substituted" case may be exemplified by, but not limited to, a $C_{1-6}$ alkoxy group, an aryloxy group, an aryl $C_{1-3}$ oxy group, a cyano group, a carboxyl group, an amino group, a $C_{1-6}$ alkoxycarbonyl group, a $C_{2-7}$ acylamide group, a tri-$C_{1-6}$ alkylsiloxy group, a siloxy group, or a silylamino group, or may be exemplified by an acetalized formyl group, a formyl group, or a halogen atom, such as chlorine or fluorine. In this context, for example, the expression "$C_{1-6}$" means 1 to 6 carbon atoms and is used with the same meaning in the following description. Further, an unsubstituted or substituted linear or branched alkyl moiety having 1 to 12 carbon atoms in the unsubstituted or substituted linear or branched alkylcarbonyl group having 1 to 24 carbon atoms may be selected with reference to the examples, and an alkyl moiety having 13 or more carbon atoms may be, for example, a tridecyl group, a tetradecyl group, a pentadecyl group, a nonadecyl group, a docosanyl group, or a tetracosyl group. In addition, the substituent may be a group containing a target site-binding moiety.

Symbol "z" is set so that, when the uPIC is formed, the total positive charge quantity derived from the side chains of the cationic poly(amino acid) segment of the block copolymer is less than 90% or more than 110% of the total negative charge quantity derived from the nucleic acid. In addition, "z" may be set so that the binding constant ($K_a$) for the nucleic acid is equal to or higher than a predetermined value. When "z" represents, for example, an integer of 22.5% or more, 25% or more, or 27% or more, which may vary depending on the value of "w", the negative charge number of the nucleic acid, and the like, with respect to the negative charge quantity of the nucleic acid, a block copolymer having a binding constant ($K_a$) equal to or higher than a predetermined value for the nucleic acid can be suitably obtained. Similarly, when "z" represents an integer of 9 or more, for example, an integer of 10 or more or an integer of 11 or more, which may vary depending on the value of "w", the negative charge number of the nucleic acid, and the like, a block copolymer having a binding constant ($K_a$) equal to or higher than a predetermined value for the nucleic acid can be suitably obtained. The upper limit of "z" may be, for example, an integer of 40 or less or an integer of 30 or less.

Symbol "w" preferably represents an integer of from 1 to 3 from the viewpoint of increasing the binding constant

for the nucleic acid, and further, more preferably represents 3 from the viewpoint of the ease of production. For example, when "z" represents 14 or less, in particular, 13 or less, "w" preferably represents an integer of from 1 to 3, for example, 3. For "w", the same group may be selected for all repeating units including these groups, or different groups may be selected for the respective repeating units. When different groups are selected, the respective repeating units are bound to each other in any suitable order, and may form a random structure or a block structure.

Symbols x1 to x4, which represent the numbers of repeats of ethylene glycol, each independently represent, as a lower limit, for example, 600, or for example, 700, or for example, 800, and as an upper limit, for example, 1,400, or for example, 1,200, or for example, 1,100.

[A-1-3. Nucleic Acid]

Herein, the nucleic acid means a poly- or oligonucleotide including nucleotides formed of a purine or pyrimidine base, a pentose, and phosphoric acid. For example, a double-stranded nucleic acid, such as double-stranded oligo- or polyRNAs, double-stranded oligo- or polyDNAs, or double-stranded oligo- or poly(nucleic acid)s in which RNA and DNA exist in a mixed state in the same strand, may be preferably used. The nucleotide contained in the nucleic acid may be of a natural type or of a chemically modified non-natural type, or may have added thereto an amino group, a thiol group, a fluorescent compound, or any other molecule.

The strand length of the nucleic acid (in the case of a double-stranded nucleic acid, the length of the part constituting the double strand) may be typically from 10-base length to 50-base length, for example, from 10-base length to 30-base length.

Preferred examples of the nucleic acid may include functional nucleic acids, such as siRNA, miRNA mimic, shRNA, and a decoy nucleic acid.

As the siRNA, for example, there may be used all ones designed for a target gene or polynucleotide by any appropriate method. Regarding the strand length of the siRNA, the part constituting a double strand may have a length of preferably from 15 bases to 50 bases, more preferably from 18 bases to 30 bases, compounds known in the art and all nucleotides having actions or functions similar to those of the compounds are encompassed. Specific examples of the siRNA include, but not limited to, ones that may be designed with reference to a gene to be targeted by gene therapy.

[A-2. Second Embodiment]

According to a second embodiment of the present invention, there is provided a unit-type polyion complex including: a specific number of molecules of a block copolymer having a poly(ethylene glycol) segment and a cationic poly(amino acid) segment; and a specific number of molecules of a nucleic acid,

wherein a total positive charge quantity derived from side chains of the cationic poly (amino acid) segment of the block copolymer is prevented from being counterbalanced with a total negative charge quantity derived from the nucleic acid,

wherein the nucleic acid has a strand length of from 10-base length to 50-base length,

wherein the poly(ethylene glycol) segment has a molecular weight of $40\times10^3$ or more, and

wherein the block copolymer has a predetermined binding constant ($K_a$) for a double-stranded nucleic acid formed of a sense strand set forth in SEQ ID NO: 1 and an antisense strand set forth in SEQ ID NO: 2, in which the 5' end of each of the strands has no phosphate group added thereto.

The uPIC according to this embodiment is such that a total positive charge quantity derived from side chains of the cationic poly(amino acid) segment of the block copolymer in a physiological pH environment is prevented from being counterbalanced with a total negative charge quantity derived from the nucleic acid, and as a result, the uPIC is not electrically neutralized. The total positive charge quantity derived from the side chains of the cationic poly(amino acid) segments of all block copolymer molecules in the uPIC in a physiological pH environment may be 45% or more and less than 90% (e.g., from 50% to 85%, or from 50% to 80%), or more than 110% and 150% or less (e.g., from 115% to 140%) of the total negative charge quantity derived from all nucleic acid molecules in the uPIC.

The block copolymer may have a binding constant ($K_a$) of, for example, $3.0\times10^5$ or more, preferably $5.0\times10^5$ or more (e.g., $8.0\times10^5$ or more, $1.0\times10^6$ or more, $1.5\times10^6$ or more, or $2.0\times10^6$ or more) for the double-stranded nucleic acid formed of the sense strand set forth in SEQ ID NO: 1 and the antisense strand set forth in SEQ ID NO: 2, in which the 5' end of each of the strands has no phosphate group added thereto. The binding constant ($K_a$) for the double-stranded nucleic acid may be set to, for example, $4.0\times10^6$ or less. Such block copolymers exhibit high affinities for short-strand nucleic acids of from about 10-base length to about 50-base length in general, and can suitably associate therewith to form uPICs. As the block copolymer, for example, the block copolymer described in the section A-1-2 may be used.

As the nucleic acid, for example, the nucleic acid described in the section A-1-3 may be used. In the uPIC according to this embodiment, a single-stranded nucleic acid (e.g., single-stranded oligo- or polyRNAs, single-stranded oligo- or polyDNAs, or single-stranded oligo- or poly (nucleic acid) s in which RNA and DNA exist in a mixed state) may be preferably used as well as the double-stranded nucleic acid. Specific examples of a single-stranded nucleic acid

having a strand length of from 10-base length to 50-base length may preferably include functional nucleic acids, such as micro RNAs, antisense nucleic acids, aptamers, and ribozymes. When the single-stranded nucleic acid is used, the uPIC according to the second embodiment may be a uPIC consisting of one molecule of the nucleic acid and one molecule of the block copolymer or a uPIC consisting of one molecule of the nucleic acid and two molecules of the block copolymer.

[B. Method of Preparing Unit-type Polyion Complex]

The uPIC of the present invent ion may be prepared by, for example, mixing any appropriate block copolymer and any appropriate nucleic acid in an aqueous solution buffered as needed (e.g., phosphate buffered saline or HEPES buffer).

[C. Composition for Nucleic Acid Delivery]

A composition for nucleic acid delivery of the present invention includes: the uPIC described in the section A; and a block copolymer capable of forming the uPIC, the block copolymer being in a free state. According to the composition for nucleic acid delivery of the present invention, more excellent blood retention property of the nucleic acid than that in the case of using the uPIC alone can be obtained. The reason why such effect is obtained may be presumed to be, for example, as described below. That is, in a solution containing the uPIC, dissociation and association between the nucleic acid and the block copolymer occur as a reversible reaction, and as a result, the uPIC is reversibly decomposed and reformed. Accordingly, when the block copolymer in a free state is blended in addition to the uPIC, the equilibrium can be shifted to the association side, and presumably as a result of this, the nucleic acid can be more suitably protected than in the case of the uPIC alone.

The block copolymer in a free state only needs to be one capable of forming the uPIC by replacing the block copolymer forming the uPIC. A specific example thereof is the block copolymer described in the section A-1-2, and a preferred example thereof is a block copolymer identical to the block copolymer forming the uPIC. Such block copolymer has a high affinity for the nucleic acid, and hence can suitably reassociate with the nucleic acid even in the bloodstream.

The composition for nucleic acid delivery of the present invention may be obtained by mixing the block copolymer and the nucleic acid in an aqueous solution, which is buffered as needed, so as to achieve a desired N/P ratio. Herein, the N/P ratio means a ratio between the molar concentration of amino groups derived from the block copolymer and the molar concentration of phosphate groups derived from the nucleic acid in the composition.

In the composition for nucleic acid delivery of the present invention, the N/Pratio is, for example, 1 ormore, preferably 1.5 or more, more preferably 2 or more. In addition, the upper limit of the N/P ratio may be set to typically 20 or less, for example, 10 or less, 5 or less, or 4 or less. In the composition for nucleic acid delivery of the present invention, when a double-stranded nucleic acid is selected as the nucleic acid to be delivered and a block copolymer having a binding constant ($K_a$) of, for example, $8.0\times10^5$ or more, preferably $1.0\times10^6$ or more for the nucleic acid is used, even if the N/P ratio is 5 or less (e.g., about 3), a blood retention time of the nucleic acid for 10% of dose or more can be prolonged to 3 hours after intravenous administration.

Examples

Now, the present invention is more specifically described by way of Examples. However, the present invention is not limited to Examples below. In Examples below, a polymer structure is described in the order of the molecular weight (kDa) of PEG and the polymerization degree of a poly (amino acid) . For example, when the molecular weight of each chain of a 2-chain branched PEG is 37 kDa and a cationic poly(amino acid) segment is formed of ten ornithine residues, the polymer is abbreviated as "PEG-pOrn(37×2-10)".

<Preparation of Block Copolymer>

With the use of 2-chain branched poly(ethylene glycol) derivatives having various molecular weights (manufactured by NOF Corporation), white powders of the following block copolymers (hydrochlorides) were obtained by the same method as a method of preparing a block copolymer described in Examples of Patent Literature 1: PEG-pOrn(37×2-11), PEG-pOrn(37×2-14), PEG-pOrn(37×2-20), PEG-pOrn(37×2-40), PEG-pLys(37×2-9), PEG-pLys(37×2-14), PEG-pLys(37×2-20), and PEG-pLys (37×2-40). The polymerization degrees of their poly(amino acid) segments were determined by $^1H$-NMR.

[Example 1]

PEG-pOrn (37×2-11) and siRNA were separately dissolved in 10 mM HEPES buffer (pH 7.3), and the resultant solutions were mixed so as to achieve a final concentration of the siRNA of 10 μM, and to achieve an N/P ratio of 0.25, 0.5, 1, 2, 3, 5, or 10, to thereby prepare PIC solutions. As the siRNA, siLuc (siRNA against luciferase) having no phosphate group added to the 5' end of each strand was used, and as required, the 5' end was labeled with Alexa647 or the like before use.

Base Sequence of siLuc:

| | |
|---|---|
| Sense | 5'-CUUACGCUGAGUACUUCGAdTdT-3' (SEQ ID NO: 1) |
| Antisense | 5'-UCGAAGUACUCAGCGUAAGdTdT-3' (SEQ ID NO: 2) |

[Example 2]

PIC solutions were prepared in the same manner as in Example 1 except that PEG-pOrn(37×2-14) was used in place of PEG-pOrn(37×2-11).

[Example 3]

PIC solutions were prepared in the same manner as in Example 1 except that PEG-pLys(37×2-14) was used in place of PEG-pOrn(37×2-11).

[Comparative Example 1]

PIC solutions were prepared in the same manner as in Example 1 except that PEG-pLys(37×2-9) was used in place of PEG-pOrn(37×2-11).

[Reference Example 1]

PIC solutions were prepared in the same manner as in Example 1 except that PEG-pLys(37×2-20) was used in place of PEG-pOrn(37×2-11). The uPIC solution (N/P ratio = 10) obtained in Reference Example 1 is substantially equivalent to the uPIC solution disclosed as the pharmaceutical preparation B in Table 2 of Patent Literature 1, and contains a uPIC consisting of one molecule of the siRNA and two molecules of the block copolymer.

<Structural Analysis of uPIC 1>

The PIC solutions prepared in Examples 1 to 3 and Comparative Example 1 described above were each subjected to analytical ultracentrifugation according to a sedimentation equilibriummethod (SE-AUC analysis) and fluorescence correlation spectroscopy analysis (FCS analysis) to determine the nucleic acid association number and block copolymer association number per PIC. Specific methods are as described below.

[SE-AUC Analysis]

The SE-AUC analysis was carried out using Beckman Optima XL-A (manufactured by Beckman Coulter), which is an ultracentrifugation system for analysis that includes an absorbance measurement system. The PIC solutions prepared at an N/P ratio of 1 in Examples and Comparative Example described above were each diluted with 10 mM HEPES buffer (pH 7.4) containing 150 mM NaCl so as to have an siRNA concentration of 0.6 μM, thereby providing measurement samples. The measurement samples were each centrifuged at 20°C for 72 hours, and then absorption at a wavelength of 260 nm was measured as a function of r. The measurement result of each of the samples was analyzed with ORIGIN software to determine the molecular weight (Da) of the PIC.

[FCS Analysis]

The FCS analysis was carried out using a confocal laser scanning microscope (manufactured by Carl Zeiss, product name "LSM510") equipped with a 40× objective lens (C-Apochromat, manufactured by Carl Zeiss) and a ConfoCor3 module. The PIC solutions prepared at an N/P ratio of 1 in Examples and Comparative Example described above were each diluted with 10 mM HEPES buffer (pH 7.4) containing 150 mM NaCl so as to have an siRNA concentration

of 100 nM, thereby providing measurement samples. The measurement samples were transferred to an 8-well chamber and subjected to FCS measurement with a sampling time of 15 seconds. The measurement was repeated 5 times. The measurement results were analyzed using the ConfoCor3 software to determine the average number of fluorescent particles within a measurement area. Subsequently, the nucleic acid association number per PIC was determined through calculation by using the following equation (a).

$$AN_{siRNA} = N_{siRNA}/N_{PIC} \quad (a)$$

In the equation, $AN_{siRNA}$ represents the nucleic acid association number per PIC, and $N_{siRNA}$ and $N_{PIC}$ represent the average number of fluorescent particles of a control sample containing only fluorescence-labeled siRNA and the average number of fluorescent particles of a measurement sample derived from a PIC solution, respectively.

The molecular weight (Da) of the PIC and the nucleic acid association number per PIC were substituted into the following equation (b) to calculate the block copolymer association number per PIC.

$$AN_{copolymer} = [MW_{PIC} - (MW_{siRNA} \times AN_{siRNA})]/MW_{copolymer} \quad (b)$$

In the equation, $AN_{copolymer}$ represents the block copolymer association number per PIC, $MW_{PIC}$ represents the molecular weight (Da) of the PIC obtained in the SE-AUC analysis, and $MW_{siRNA}$ and $MW_{copolymer}$ represent the molecular weights (Da) of the siRNA and the block copolymer calculated on the basis of their structural formulae, respectively.

The results of the structural analysis are shown in Table 1.

Table 1

| | Polymer used | Molecularweight of PIC (kDa) | siRNA association number | Polymer association number | Charges of polymer side chains/nucleic acid charges* (%) |
|---|---|---|---|---|---|
| Example 1 | PEG-pOrn (37×2-11) | 180 | 1.0±0.1 | 2.210.1 | 55 |
| Example 2 | PEG-pOrn (37×2-14) | 148 | 1.0±0.1 | 1.8±0.1 | 70 |
| Example 3 | PEG-pLys (37×2-14) | 160 | 1.0±0.1 | 1.9±0.1 | 70 |
| Comparative Example 1 | PEG-pLys (37×2-9) | 169 | 1.1±0.1 | 2.0±0.1 | 45 |
| * : The ratio of charges derived from polymer side chains to charges derived from the nucleic acid calculated assuming that the nucleic acid association number per PIC is 1 and the polymer association number is 2 | | | | | |

As shown in Table 1, the siRNA association number and the block copolymer association number determined by actually performing FCS and SE-AUC analyses are about 1 and about 2, respectively. In this connection, the fact that the standard deviation of each association number is 0.1 or less shows that the PICs obtained in Examples 1 to 3 and Comparative Example 1 each have extremely high structural homogeneity and have a specific uniform structure. In view of the foregoing, it is logically understood that the PICs prepared in Examples 1 to 3 and Comparative Example 1 are each a uPIC consisting of one molecule of siRNA and two molecules of block copolymer.

<Structural Analysis of uPIC 2>

The uPIC solutions obtained in Reference Example 1 described above were subjected to FCS analysis in the same manner as in the "Structural Analysis of uPIC 1" to determine the nucleic acid association number per PIC and the average particle diameter (hydrodynamic diameter: $D_H$) of the PIC. The average particle diameter ($D_H$) of the PIC was calculated with ConfoCor3 by: converting an autocorrelation curve obtained in the FCS measurement into a diffusion time; then converting the diffusion time into a diffusion coefficient ($D_C$) with the use of a Cy5 dye as a control; and substituting the diffusion coefficient into Einstein's relationship (Stokes-Einstein equation). The results are shown in FIG.

2.

As shown in FIG. **2,** the uPIC solutions of Reference Example 1 had average particle diameters of the uPIC of about 18 nm and nucleic acid association numbers of about 1, both of which were nearly constant over the N/P ratio range of from 1 to 10. In view of this, it is understood that the uPIC of Reference Example 1 is present as a stable unit structure, without forming a secondary association between the uPICs or with a free block copolymer.

<Measurement of Binding Constant>

The binding constant ($K_a$) of each of the block copolymers obtained above for siRNA was determined by the following method. The results are shown in Table 2.

The binding constant was determined by quantifying the intensity of fluorescence enhanced by the binding of ethidiumbromide (EtBr) to the nucleic acid in competitive binding of EtBr and the block copolymer to siRNA. The fluorescent intensity was quantified using a spectrofluorometer (manufactured by JASCO Corporation, FP-6600). Specifically, the block copolymer serving as a measurement object and the siRNA (siLuc used in Examples and Comparative Example, without the Alexa647 label) were separately dissolved in 10 mM HEPES buffer (pH 7.3) containing 150 mM NaCl, and the resultant solutions were mixed so as to achieve a final concentration of the siRNA of 1 μM, and to achieve an N/P ratio of 0.25, 0.5, or 1, to thereby prepare PIC solutions. To 1 mL of the PIC solution at each N/P ratio, an about 0.5 mM aqueous solution of EtBr was added dropwise 10 times in amounts of 10 μL, and the fluorescent intensity (ex 525 nm, em 600 nm) was measured after each time of dropwise addition. The binding constant was calculated by analyzing the resultant fluorescent intensities on the basis of a method of a previous report (J. Chem. Edu. 71, 77 (1994)).

Table 2

| Block copolymer | Binding constant ($K_a$) |
|---|---|
| PEG-pOrn(37×2-11) | $1.1\times10^6$ |
| PEG-pOrn(37×2-14) | $2.3\times10^6$ |
| PEG-pOrn(37×2-20) | $2.1\times10^6$ |
| PEG-pOrn(37×2-40) | $2.3\times10^6$ |
| PEG-pLys(37×2-9) | $1.0\times10^5$ |
| PEG-pLys(37×2-14) | $4.0\times10^5$ |
| PEG-pLys(37×2-20) | $4.2\times10^5$ |
| PEG-pLys(37×2-40) | $4.2\times10^5$ |
| Reference: EtBr*1 | $6.4\times10^5$ |
| *1: value measured using naked siRNA in place of PIC | |

<Evaluation of Blood Retention Property of siRNA 1>

The uPIC solutions (N/P ratio = 1) obtained in Examples, Comparative Example, and Reference Example were administered to the tail veins of 6-week-old female BALB/c mice. At this time, the administration was carried out so that the dose was 2 nmol/mouse of siLuc. After that, fluorescent intensities attributed to siLuc in the blood vessels in the ears of the mice were measured over time using an in vivo confocal imaging system (NikonA1R, manufactured by Nikon Corporation), and relative fluorescent intensities were determined according to the following equation ($n$ = 1). Relative fluorescent intensities obtained immediately after the administration, after 5 minutes, after 10 minutes, after 30 minutes, and after 60 minutes are shown in Table 3.

Relative fluorescent intensity (%) = (intensity at the measurement time - background intensity)/(maximum intensity - background intensity)×100

Table 3

| | Polymer used | Relative fluorescent intensity (%) | | | | |
|---|---|---|---|---|---|---|
| | | Immediately after administration | After 5 minutes | After 10 minutes | After 30 minutes | After 60 minutes |
| Example 1 | PEG-pOrn (37×2 -11) | 100 | 96 | 84 | 82 | 58 |
| Example 2 | PEG-pOrn (37×2-14) | 100 | 96 | 84 | 63 | 34 |
| Example 3 | PEG-pLys (37×2-14) | 100 | 78 | 66 | 27 | 14 |
| Comparative Example 1 | PEG-pLys (37×2 -9) | 100 | (Uneval uable) | (Uneval uable) | (Uneval uable) | (Uneval uable) |
| Reference Example 1 | PEG-pLys (37×2 -20) | 100 | 73 | 48 | 23 | 9 |

As shown in Table 3, the uPIC solutions of Examples 1 to 3 each provided excellent blood retention property of the nucleic acid, which was comparable to or higher than that of the uPIC solution of Reference Example 1. In particular, in each of Examples 1 and 2 using the block copolymer having a binding constant ($K_a$) for the nucleic acid of more than $1.0\times10^6$, more excellent blood retention property of the nucleic acid than that of Reference Example 1 was obtained. Meanwhile, the uPIC solution of Comparative Example 1 showed a significant reduction in fluorescent intensity immediately after the administration, and stable measurement was impossible thereafter.

<Evaluation of Blood Retention Property of siRNA 2>

In the same manner as in Evaluation of Blood Retention Property of siRNA 1 except for using the uPIC solutions at an N/P ratio of 10, the uPIC solutions were administered to the tail veins of 6-week-old female BALB/c mice, fluorescent intensities attributed to siLuc in the bloodstream in the ears of the mice were measured over time, and relative fluorescent intensities were determined ($n$ = 1). Changes over time in relative fluorescent intensity are shown in FIG. **3.**

As shown in FIG. **3,** the uPIC solutions (compositions for nucleic acid delivery) of Examples 1 to 3 each provided excellent blood retention property of the nucleic acid, which was comparable to that of the uPIC solution of Reference Example 1. Meanwhile, the uPIC solution of Comparative Example 1 was inferior in blood retention property of the nucleic acid to the uPIC solutions of Examples and Reference Example 1.

Industrial Applicability

The uPIC of the present invention can be suitably applied in drug delivery systems for nucleic acid therapeutics and the like.

Reference Signs List

**100** unit-type polyion complex

**10** block copolymer
**12** cationic poly(amino acid) segment
**13, 14** poly(ethylene glycol) segment
**20** nucleic acid

## Claims

1. A unit-type polyion complex, comprising:

    a specific number of molecules of a block copolymer having a poly(ethylene glycol) segment and a cationic poly(amino acid) segment; and
    a specific number of molecules of a nucleic acid,
    wherein a total positive charge quantity derived from side chains of the cationic poly (amino acid) segment of the block copolymer in the unit-type polyion complex is prevented from being counterbalanced with a total negative charge quantity derived from the nucleic acid,
    wherein the nucleic acid has a strand length of from 10-base length to 50-base length,
    wherein the poly(ethylene glycol) segment has a molecular weight of $40\times10^3$ or more, and
    wherein the block copolymer has a binding constant ($K_a$) for the nucleic acid of $3.0\times10^5$ or more.

2. The unit-type polyion complex according to claim 1, wherein the unit-type polyion complex consists of two molecules of the block copolymer and one molecule of the nucleic acid.

3. The unit-type polyion complex according to claim 1 or 2, wherein the nucleic acid is a double-stranded nucleic acid.

4. The unit-type polyion complex according to any one of claims 1 to 3, wherein the molecular weight of the poly(ethylene

glycol) segment is $60 \times 10^3$ or more.

**5.** The unit-type polyion complex according to any one of claims 1 to 4, wherein the poly (ethylene glycol) segment is 2-chain branched, and wherein each poly (ethylene glycol) chain of the poly (ethylene glycol) segment has a molecular weight of $20 \times 10^3$ or more.

**6.** The unit-type polyion complex according to any one of claims 1 to 5, wherein the cationic poly (amino acid) segment contains ornithine as a constituent amino acid.

**7.** A composition for nucleic acid delivery, comprising:

the unit-type polyion complex of any one of claims 1 to 6; and
a block copolymer capable of forming the unit-type polyion complex, the block copolymer being in a free state.

**8.** The composition for nucleic acid delivery according to claim 7, wherein a ratio (N/P ratio) between a molar concentration of amino groups derived from the block copolymer and a molar concentration of phosphate groups derived from the nucleic acid in the composition is from 1 to 20.

FIG. 1

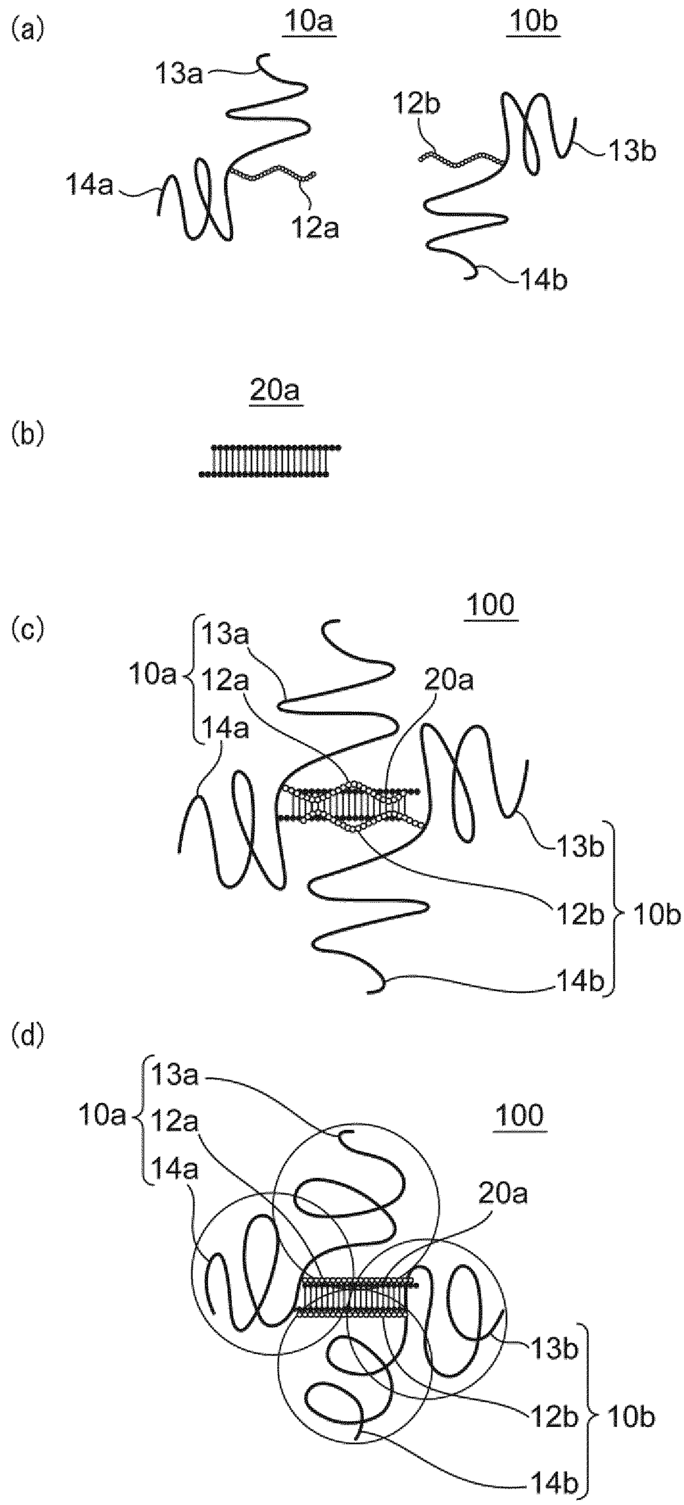
(a)
10a
10b
13a
12b
13b
14a
12a
14b
20a
(b)
(c)
100
10a
13a
12a
20a
14a
13b
12b
10b
14b
(d)
13a
10a
12a
100
14a
20a
13b
12b
10b
14b

FIG. 2

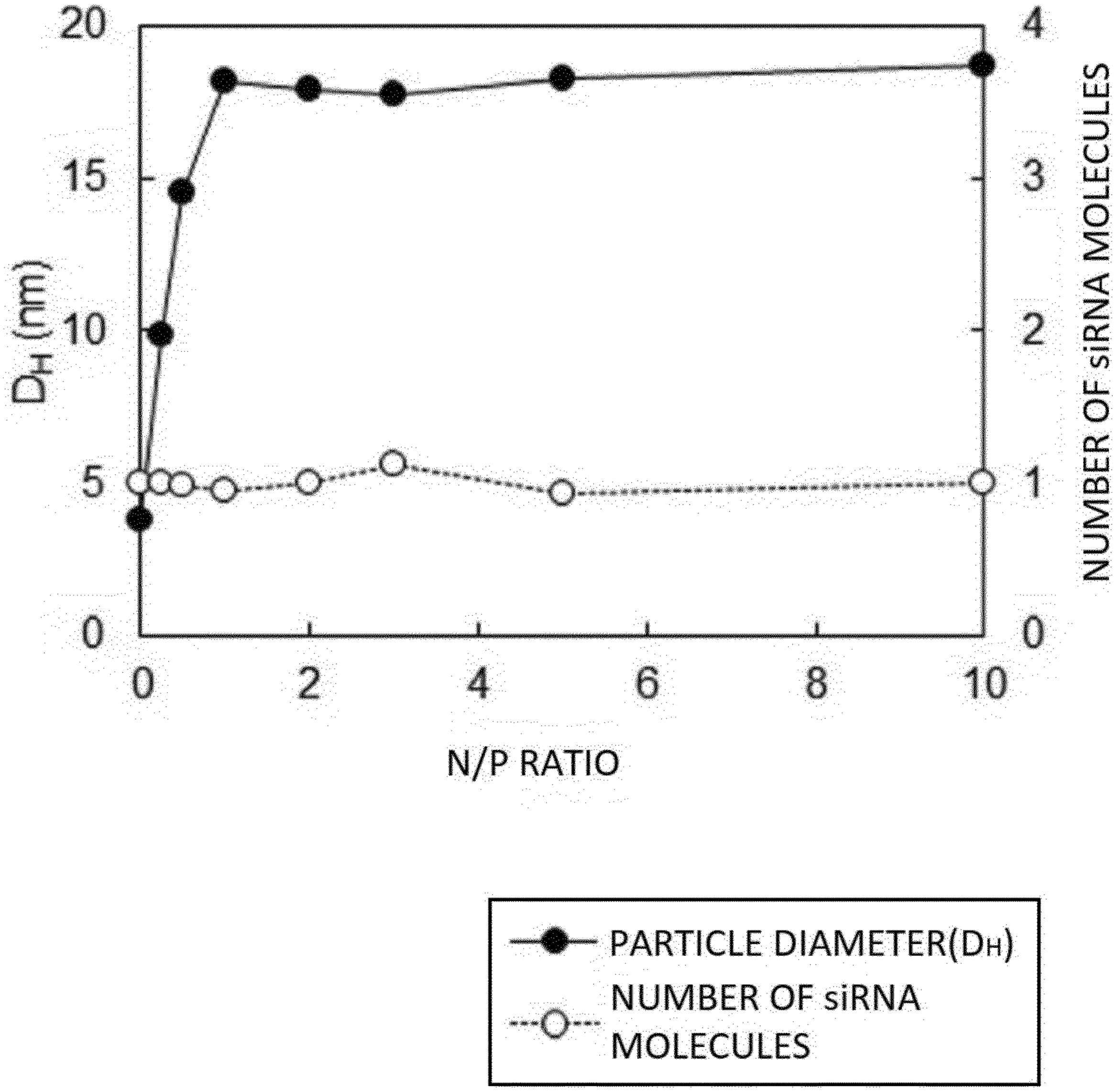
20
15
10
5
0
$D_H$ (nm)
4
3
2
1
0
NUMBER OF siRNA MOLECULES
0
2
4
6
8
10
N/P RATIO
PARTICLE DIAMETER($D_H$)
NUMBER OF siRNA MOLECULES

FIG. 3

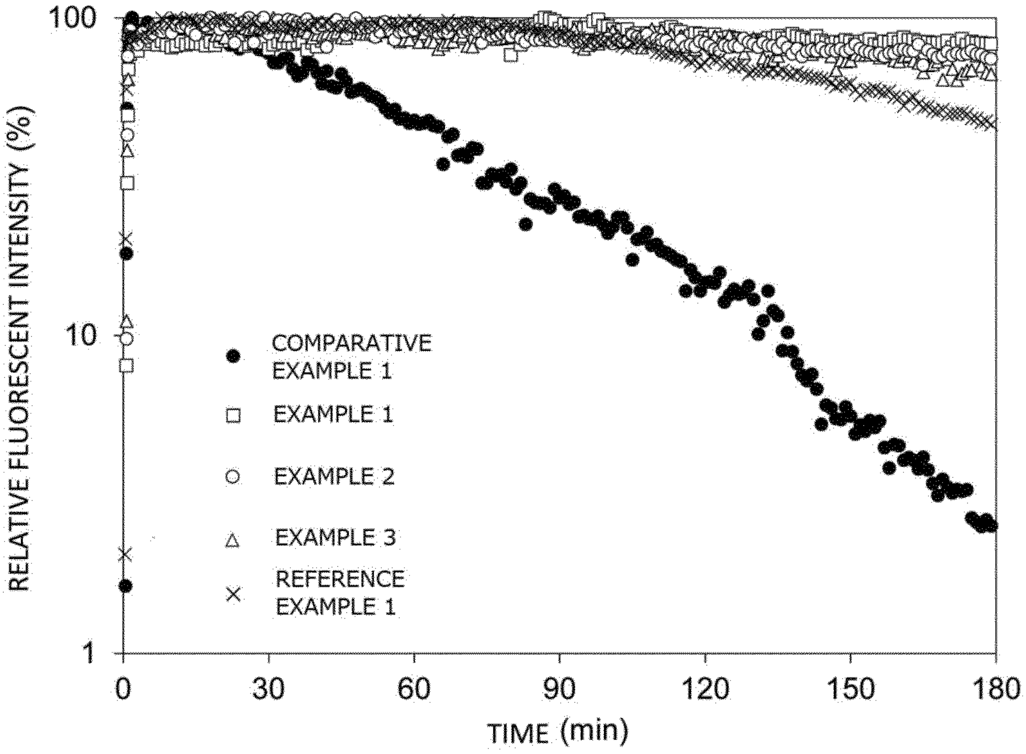
100
10
1
RELATIVE FLUORESCENT INTENSITY (%)
0
30
60
90
120
150
180
TIME (min)
COMPARATIVE EXAMPLE 1
EXAMPLE 1
EXAMPLE 2
EXAMPLE 3
REFERENCE EXAMPLE 1

## INTERNATIONAL SEARCH REPORT

International application No.
PCT/JP2018/032040

A. CLASSIFICATION OF SUBJECT MATTER
Int.Cl. A61K31/713(2006.01)i, A61K9/10(2006.01)i, A61K47/34(2017.01)i, A61K48/00(2006.01)i, A61P35/00(2006.01)i, C12N15/09(2006.01)i

According to International Patent Classification (IPC) or to both national classification and IPC

B. FIELDS SEARCHED

Minimum documentation searched (classification system followed by classification symbols)
Int.Cl. A61K31/713, A61K9/10, A61K47/34, A61K48/00, A61P35/00, C12N15/09

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)
CAplus/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| X | WO 2013/162041 A1 (THE UNIVERSITY OF TOKYO) 31 October 2013, paragraphs [0010], [0017], [0046]-[0047], table 1 & US 2015/0080454 A1, paragraphs [0022], [0031], [0076]-[0077], table 1 & EP 2842546 A1 | 1-8 |
| A | WO 2006/123631 A1 (KYUSHU UNIVERSITY) 23 November 2006, paragraphs [0094]-[0096], table 1 & JP 2008-201673 A | 1-8 |

☒ Further documents are listed in the continuation of Box C.    ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 November 2018 (05.11.2018) | 20 November 2018 (20.11.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No. PCT/JP2018/032040

C (Continuation). DOCUMENTS CONSIDERED TO BE RELEVANT

| Category* | Citation of document, with indication, where appropriate, of the relevant passages | Relevant to claim No. |
|---|---|---|
| A | DEROUCHEY, J. et al., "Monomolecular Assembly of siRNA and Poly (ethylene glycol)-Peptide Copolymers", Biomacromolecules, 2008, vol. 9, no. 2, pp. 724-732, section "Synthesis of copolymers." of "Materials and Methods" | 1-8 |
| A | WO 2005/078084 A1 (TODAI TLO, LTD.) 25 August 2005, example 1 (Family: none) | 1-8 |
| P, A | WO 2018/079841 A1 (GIFU UNIVERSITY) 03 May 2018, test example 7 (Family: none) | 1-8 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013162041 A1


### Non-patent literature cited in the description


- *J. Chem. Edu.,* 1994, vol. 71, 77

SEQUENCE LISTING

```
<110>  The University of Tokyo
        Innovation Center of NanoMedicine

<120>  Unit type polyion complex carrying a nucleic acid

<130>  TKU15218PCT

<150>  JP2017-166735
<151>  2017-08-31

<160>  2

<170>  PatentIn version 3.5

<210>  1
<211>  21
<212>  DNA
<213>  Artificial Sequence

<220>
<223>  sense strand of siRNA for luciferase including dT terminus

<400>  1
cuuacgcuga guacuucgat t                                                21


<210>  2
<211>  21
<212>  DNA
<213>  Artificial Sequence

<220>
<223>  antisense strand of siRNA for luciferase including dT terminus

<400>  2
ucgaaguacu cagcguaagt t                                                21
```